# EUROPÄISCHE PATENTSCHRIFT

(11) **EP 0 388 778 B1**
(45) Veröffentlichungstag und Bekanntmachung des Hinweises auf die Patenterteilung: **10.01.1996**
(21) Anmeldenummer: 90104859.5
(22) Anmeldetag: 15.03.1990
(51) Int. Cl.: C07D 317/20

(54) **Herstellung von Dioxolanderivaten**
Preparation of dioxolane derivatives
Préparation de dérivés de dioxolanes

(30) Priorität: 23.03.1989 CH 1093/89
(43) Veröffentlichungstag der Anmeldung: 26.09.1990
(73) Patentinhaber: F. HOFFMANN-LA ROCHE AG, CH-4002 Basel (CH)
(72) Erfinder: Barner, Richard, Dr., CH-4108 Witterswil (CH); Hübscher, Josef, CH-4208 Nunningen (CH); Wirz, Beat, Dr., CH-4153 Reinach (CH)
(74) Vertreter: Urech, Peter, Dr.

(56) Entgegenhaltungen:
- EP-A- 0 244 912
- TETRAHEDRON, (INCL. TETRAHEDRON REPORTS) Bd. 44, Nr. 9, 1988, OXFORD GB Seiten 2575-2582
- JOURNAL OF THE CHEMICAL SOCIETY, Nr. 8, 1987, LETCHWORTH GB, Seiten538-539
- COLLECTION OF CZECHOSLOVAK CHEMICAL COMMUNICATIONS, Bd. 54, Nr. 1, Januar 1989, PRAGUE CS, Seiten 248-265

## Beschreibung

Die vorliegende Erfindung betrifft ein Verfahren zur Herstellung von Zwischenprodukten, und zwar von chiralen Dioxolanderivaten der allgemeinen Formeln 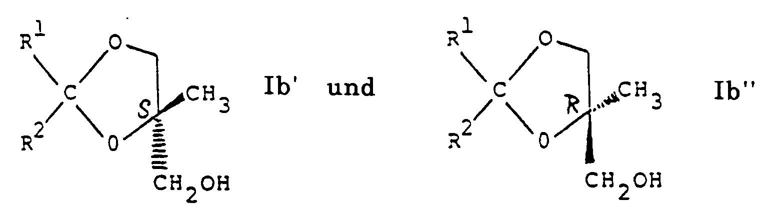
worin R¹ und R² unabhängig voneinander Methyl oder Aethyl, oder zusammen Pentamethylen bedeuten,
sowie die Verwendung dieser Zwischenprodukte zur Herstellung von d-α-Tocopherol.

Die Verbindungen der Formeln Ib' und Ib'' sind zum Teil neu und sind wertvolle Zwischenprodukte bei der Herstellung von Vitamin E, insbesondere von dessen optisch aktiver Form d-α-Tocopherol.

Die europäische Patentpublikation 244 912 offenbart ein Verfahren zur Herstellung von {(R)-2,2- un- oder disubstituiertem 1,3-dioxolan-4-yl} methanol, indem man das entsprechende racemische Methanol der Einwirkung eines Mikroorganismus oder einer davon derivierten Substanz unterwirft, der bzw. die dazu fähig ist, das (S)-Methanol stereoselektiv aufzunehmen. Das aufgenommene (S)-Methanol kann in die entsprechende (R)-4-Carbonsäure übergeführt werden. Bei diesem Verfahren handelt es sich nicht um eine enzymatische Hydrolyse. Ferner weisen das Edukt und die Produkte (Dioxolanderivate) jeweils nur einen Substituenten in der 4-Stellung des Dioxolankerns auf.
Tetrahedron 44, Nr. 9,2575 - 2582 (1988) offenbart die Hydrolyse von 0-Phenylacetaten verschiedener 2,2-Dimethyl-1,3-dioxolan-4-methanole, welche gegebenenfalls weitere Substituenten in den 4- und 5-Stellungen aufweisen, unter Verwendung einer immobilisierten Penicillinase als Enzym. Auf diese Weise wird beispielsweise das {(S)-2,2,4-Trimethyl-1,3-dioxolan-4-yl}-methanol mit einer Enantiomerenreinheit (ee) von 0,9 erhalten. Sowohl die Edukte als auch der Katalysator (Enzym) unterscheiden sich von denjenigen des erfindungsgemässen Verfahrens.

Das erfindungsgemässe Verfahren ist dadurch gekennzeichnet, dass man einen Alkansäureester der allgemeinen Formel 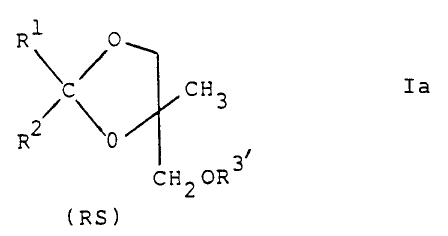
worin
R¹ und R² die oben angegebenen Bedeutungen besitzen und
R^{3'} C₂₋₉-Alkanoyl bedeutet,
unter Verwendung eines Enzyms der Unterklasse Carboxylesterasen (EC 3.1.1.1), Triacylglycerinlipasen (EC 3.1.1.3), Cholesterinesterasen (EC 3 1.1.13) oder Diacylglycerinlipasen (EC 3.1.1.34) zur Verbindung der allgemeinen Formel Ib' hydrolysiert bzw.
den bei der Durchführung der enzymatischen Hydrolyse nicht umgesetzten (S)-Alkansäureester von Ia zum entsprechenden (R)-Alkohol der allgemeinen Formel Ib' hydrolysiert.

Die enzymatische Hydrolyse wird erfindungsgemäss unter Einwirkung eines Enzyms der oben angegebenen Unterklassen durchgeführt, und zwar vorzugsweise einer Lipase mikrobieller Herkunft aus Pseudomonas fluorescens, Mucor miehei, Rhizopus delemar, Rhizopus arrhizus, Humicola lanuginosa, Rhizopus javanicus oder Mucor javanicus. Beispiele geeigneter Enzympräparate sind Lipase P-30 (P. fluorescens; Amano Pharmaceutical Co. Ltd., Nagoya, Japan), Lipase SAM (P. fluorescens; Amano; unter der Katalog-Nr. 62312 von Fluka AG, Buchs, Schweiz erhältlich), Lipase PM und LMM (M. miehei; Palatase M 1000L bzw. Lipozyme IM 20; Novo Industri A/S, Bagsvaerd, Dänemark), Lipase LRD und D (R. delemar; Seikagaku Kogyo Co. Ltd., Tokyo, Japan bzw. Amano), Lipase LRA (R. arrhizus; Sigma Chemical Co., St. Louis, USA), Lipase CF (H. lanuginosa; Amano), Lipase FAP (R. javanicus: Amano) und Lipase MAP (M. javanicus; Amano).

Die Enzyme können sowohl in partiell oder vollgereinigtem Zustand als auch in immobilisiertem Zustand eingesetzt werden.

Das erfindungsgemässe Verfahren wird zweckmässigerweise in einer organisch/wässrigen Emulsion durchgeführt, in der die organische Phase allein aus dem Ausgangsmaterial der Formel Ia bestehen kann, gegebenenfalls aber auch ein mit Wasser nicht mischbares, unpolares oder polares Lösungsmittel, wie beispielsweise n-Hexan, Isooktan oder Diäthyläther, enthält. In der wässrigen Phase kann einer der üblichen anorganischen und organischen Puffer, z.B. Natriumphosphat oder Natriumcitrat, vorhanden sein, und zwar zweckmässigerweise bei einer molaren Konzentration von 1mM bis 1,0M, vorzugsweise 3mM bis 0,1M.

Zudem kann das erfindungsgemässe Verfahren in Gegenwart eines mono- oder polyhydrischen Alkohols, z.B. Aethanol oder Glycerin, und zwar zweckmässigerweise bei einer molaren Konzentration von 50mM bis 4M, vorzugsweise 50mM bis 0,5M; von Calcium- oder Magnesiumionen; eines handelsüblichen Emulgators, z.B. Polyvinylalkohol oder Triton® X-100; und/oder eines "Einsalz"-Salzes, z.B. Lithiumrhodanid oder Guanidiniumchlorid, und zwar zweckmässigerweise bei einer molaren Konzentration von 50mM bis 2M, vorzugsweise 50mM bis 0,5M, durchgeführt werden.

Die Konzentration des Substrats (Alkanesters der Formel Ia) im gesamten Reaktionsmedium ist zweckmässigerweise 0,5% bis 50%, vorzugsweise 1% bis 15%, wobei die prozentualen Konzentrationen sich auf Gewicht/Volumen (G/V) beziehen. Das Gewichtsverhältnis Enzym:Substrat beträgt zweckmässigerweise 0,001-0,05:1.

Zweckmässigerweise erfolgt die erfindungsgemässe Hydrolyse bei Temperaturen zwischen 0° und 45°C, vorzugsweise zwischen 10° und 35°C. Man führt die Hydrolyse zweckmässigerweise bei einem pH-Wert von 6 bis 9, vorzugsweise 7 bis 8, durch.

Ausgehend vom racemischen (RS)-Alkansäureester Ia erhält man zunächst als Reaktionsprodukt überwiegend den (S)-Alkohol der Formel Ib', d.h. es handelt sich bei dem erfindungsgemässen Verfahren um eine asymmetrische Hydrolyse, indem sich der (R)-Ester des racemischen Gemisches Ia unter der Einwirkung des Enzyms viel leichter zum (S)-Alkohol Ib' hydrolysieren lässt als der (S)-Ester zum (R)-Alkohol der Formel 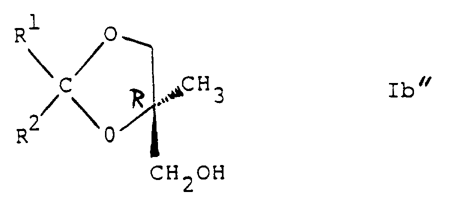
Dabei bleibt bis zu einem 50-prozentigen Reaktionsumsatz der weitaus überwiegende Teil des (S)-Esters, d.h. der Formel 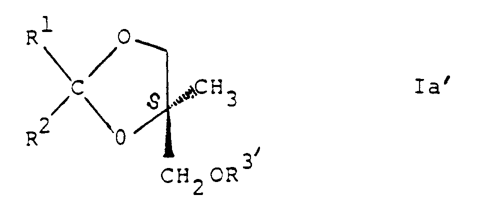
des racemischen Gemisches Ia zurück. Es wurde gefunden, dass bis zu einem 50-prozentigen Reaktionsumsatz das Verhältnis Alkohol Ib' [(S)-Alkohol]:Alkohol Ib'' [(R)-Alkohol], d.h. der sogenannte ee-Wert (enantiomerer Ueberschuss oder enantiomere Reinheit = enantiomeric excess), äusserst hoch bleibt, und zwar bei mindestens 90%, in den meisten Fällen um 98% herum. Hierin besteht der Vorteil des erfindungsgemässen Verfahrens. Ein weiterer wesentlicher Vorteil des erfindungsgemässen Verfahrens besteht darin, dass mit der Bildung des Produktes Ia' nicht etwa ein Nebenprodukt sondern ein Produkt anfällt, das ebenfalls in d-α-Tocopherol übergeführt werden kann.

Nach Erreichen des 50-prozentigen Reaktionsumsatzes, d.h. zum Zeitpunkt der Beendigung der Umwandlung des im Racemat Ia anwesenden (R)-Esters zum (S)-Alkohol Ib' (was durch konventionelle analytische Methoden, z.B. Titration, festgestellt werden kann), wird die Reaktion abgebrochen, beispielsweise durch Zugabe eines organischen Lösungsmittels, z.B. Methylenchlorid oder Methyl-tert.butyläther. Dann werden der (S)-Alkohol und der nicht umgesetzte (S)-Ester vom Reaktionsgemisch isoliert, z.B. durch Extrahieren mit einem organischen Lösungsmittel, wie beispielsweise Chloroform oder Methyl-tert.butyläther, und anschliessendes Abdampfen des Lösungsmittels, und die beiden Komponenten voneinander getrennt, z.B. durch fraktionierte Destillation oder Säulenchromatographie.

Wie oben gesagt, sind die Verbindungen der Formeln Ia' und Ib' wertvolle Zwischenprodukte bei der Herstellung des optisch aktiven d-α-Tocopherols. Wie in der obigen Definition des erfindungsgemässen Verfahrens erwähnt, wird der bei der Durchführung der enzymatischen Hydrolyse nicht umgesetzte (S)-Alkansäureester von Ia zum (R)-Alkohol der allgemeinen Formel Ib'' hydrolysiert. Die erfindungsgemässe Verwendung dieses Verfahrens zur Herstellung von d-α-Tocopherol ist dadurch gekennzeichnet, dass man den so hergestellten (S)-Alkohol der Formel Ib' bzw. (R)-Alkohol der Formel Ib'' zum entsprechenden (R)-Alkansulfonat oder (R)-Arylsulfonat verestert, die Schutzgruppe R¹R²C< abspaltet und das resultierende (R)- bzw. (S)-Diol in an sich bekannter Weise in d-α-Tocopherol überfuhrt. Die diesbezüglichen, unter Verwendung dieser Zwischenprodukte durchführbaren Synthese-Zugänge sind in den nachfolgenden Reaktionsschemata veranschaulicht: 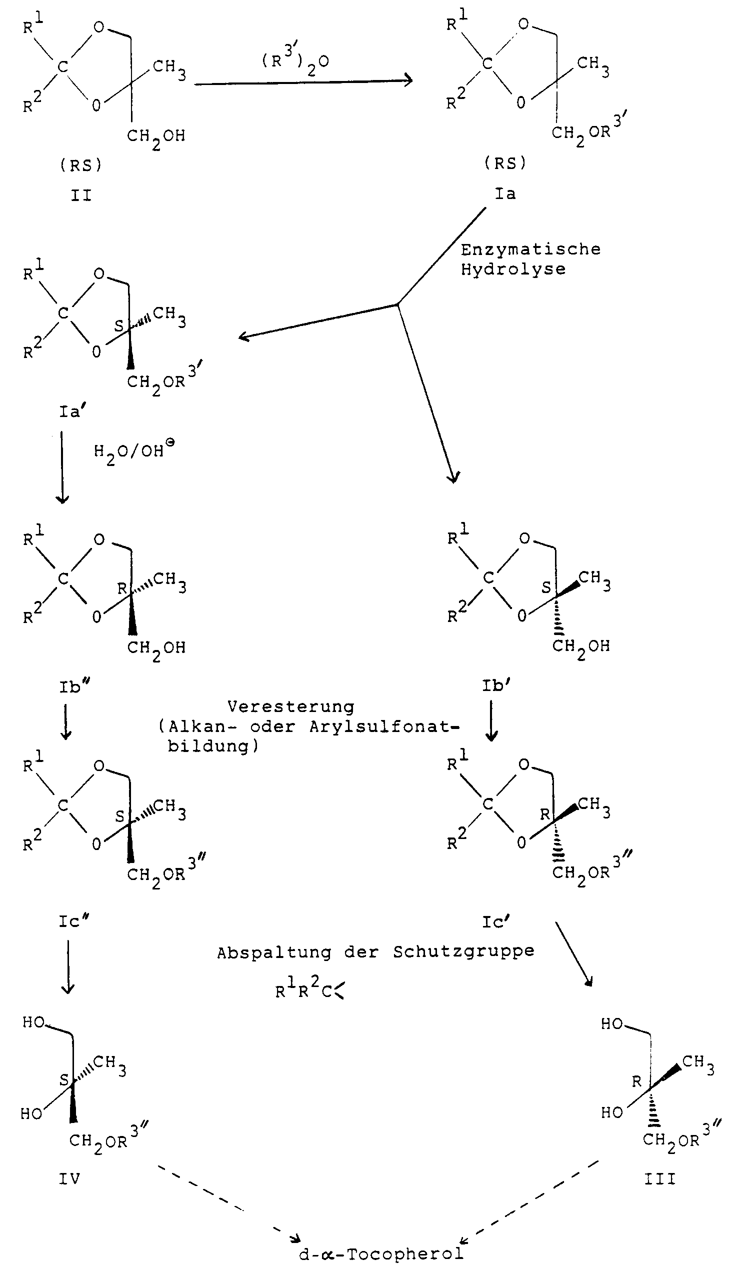
In den obigen Reaktionsschemata bedeuten R^{3'} C₂₋₉-Alkanoyl, also Acetyl bis Nonanoyl, wobei n-Butyryl bevorzugt ist; und R^{3''} Alkan- oder Arylsulfonyl, vorzugsweise Methansulfonyl (Mesyl) bzw. p-Tolylsulfonyl (Tosyl). Die Verbindungen der Formeln II und Ia treten als Racemate [(RS)-Formen] auf, während die Verbindungen der Formeln Ib'', Ic' und III in der (R)-Enantiomerenform und die Verbindungen der Formeln Ia', Ib', Ic'' und IV in der (S)-Enantiomerenform vorliegen. In diesen Formeln ist unter dem Zeichen 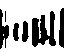 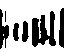 oder 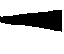 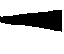 zu verstehen, dass der Substituent CH₃, CH₂OR^{3'}, CH₂OH oder CH₂OR^{3''} hinter bzw. vor der Molekülebene liegt.

Die als Ausgangsmaterialien verwendeten (RS)-Alkohole der allgemeinen Formel II sind zum Teil bekannt (siehe z.B. J. Org. Chem. 1983, 48, 3592-3594, und J. Chem. Soc., Chem. Commun., 1987, 538-539, in denen diejenigen (RS)-Verbindungen II beschrieben sind, in denen sowohl R¹ als auch R² Methyl bedeutet). Die restlichen, also neuen, (RS)-Alkohole der Formel II, d.h. diejenigen Verbindungen der Formel II, in denen R¹ und R² die oben angegebenen Bedeutungen besitzen, allerdings nicht beide Methyl bedeuten, können nach an sich bekannten Methoden hergestellt werden.

Die meisten Zwischenprodukte der Formeln Ia, Ia', Ib', Ib'', Ic' und Ic'' sind neu und bilden einen weiteren Gegenstand der vorliegenden Erfindung. Diese neuen Verbindungen werden durch die nachstehende Formel I umfasst: 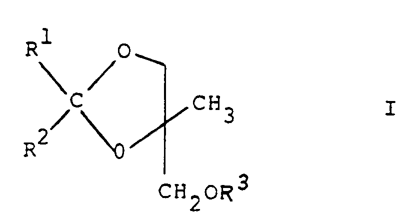
worin
- R¹ und R²: unabhängig Voneinander Methyl oder Aethyl oder zusammen Pentamethylen
und
- R³: Wasserstoff, C₂₋₉-Alkanoyl, Alkansulfonyl oder Arylsulfonyl bedeuten, wobei, falls R¹ und R² beide für Methyl stehen, R³ Verschieden von Wasserstoff oder Arylsulfonyl ist, und falls R¹ und R² zusammen für Pentamethylen und R³ für Wasserstoff stehen, die Verbindung I chiral ist.

Unter den Verbindungen der Formel I sind demgemäss sowohl die chiralen Verbindungen [(R)- oder (S)-Enantiomerenform] als auch Gemische der beiden Formen, also die Racemate, zu verstehen, und das {2-Methyl-1,4-dioxaspiro-[4,5]dec-2-yl}methanol (Formel I, in der R¹ und R² zusammen Pentamethylen und R³ Wasserstoff bedeuten) als Racemat ist ausgenommen.

Aus der Definition der Verbindungen der Formel I und den obigen Reaktionsschemata ist ersichtlich, dass die erfindungsgemässen neuen Verbindungen der Formel I aus den folgenden Unterklassen bestehen:

Die Verbindungen der allgemeinen Formel Ia, in denen R¹ und R² die oben angegebenen Bedeutungen besitzen, und R^{3'} C₂₋₉-Alkanoyl bedeutet [unter diesen Verbindungen befinden sich insbesondere die Racemate [(RS)-Form], welche die Produkte der Umsetzung der racemischen Verbindungen der allgemeinen Formel II mit dem jeweiligen Säureanhydrid (R^{3'})₂O sind, sowie die Verbindungen der allgemeinen Formel Ia', also die (S)-Enantiomeren];
die Verbindungen der allgemeinen Formel 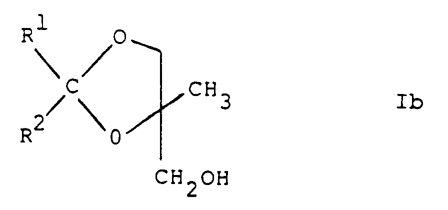
worin R¹ und R² unabhängig voneinander Methyl oder Aethyl, oder zusammen Pentamethylen bedeuten, wobei R¹ und R² nicht beide für Methyl stehen, und falls R¹ und R² zusammen für Pentamethylen stehen, die Verbindung Ib chiral ist, also entweder in der (R)-Enantiomerenform oder in der (S)-Enantiomerenform vorliegt [unter diesen Verbindungen befinden sich insbesondere Verbindungen der allgemeinen Formel Ib', also die (S)-Enantiomeren, sowie Verbindungen der allgemeinen Formel Ib'', also die (R)-Enantiomeren];
sowie die Verbindungen der allgemeinen Formel 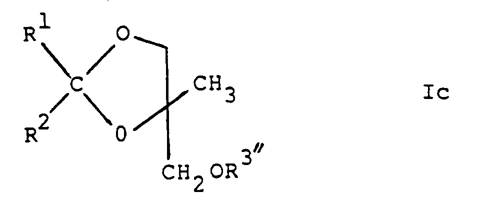
worin R¹ und R² unabhängig voneinander Methyl oder Aethyl, oder zusammen Pentamethylen, und R^{3''} Alkan- oder Arylsulfonyl bedeuten, wobei, falls R¹ und R² beide für Methyl stehen, R^{3''} verschieden von Arylsulfonyl ist,
unter denen sich insbesondere die meisten Verbindungen der allgemeinen Formel Ic', also die (R)-Enantiomeren, sowie die meisten Verbindungen der allgemeinen Formel Ic'', also die (S)-Enantiomeren, befinden.

Die Formeln Ib' und Ib'' umfassen definitionsgemäss nicht nur neue Verbindungen, sondern auch die bekannten Verbindungen der Formeln Ib' und Ib'', in denen R¹ und R² beide für Methyl stehen (siehe J. Org. Chem. 1983, 48, 3592-3594 und J. Chem. Soc. Chem. Commun., 1987, 538-539).

Die wie oben definierten Verbindungen der Formel Ic umfassen u.a. nicht das {2,2,4-Trimethyl-1,3-dioxolan-4-yl}methyltosylate, da diese Verbindung sowohl im Liebigs Ann. Chem. 1989, 9-18 als auch in Collect. Czech. Chem. Commun. Vol 54,248-265 (1989) offenbart ist.

Unter dem Ausdruck "C₂₋₉-Alkanoyl" sind sowohl geradkettige als auch verzweigte Alkanoylgruppen zu verstehen. Dies gilt ebenfalls für "Alkansulfonyl" (R^{3''}), wobei der Alkanteil insbesondere C₁₋₄-Alkyl ist. Die Arylsulfonylgruppe (R^{3'')} ist insbesondere Phenyl-, Tolyl- oder Naphthylsulfonyl. Wie bereits oben erwähnt, ist die bevorzugte C₂₋₉-Alkanoyl-, Alkansulfonyl- oder Arylsulfonylgruppe n-Butyryl, Methansulfonyl (Mesyl) resp. p-Toluolsulfonyl (Tosyl).

Besonders bevorzugte einzelne erfindungsgemässe oder im erfindungsgemässen Verfahren umgesetzte Verbindungen sind:
Formel Ia bzw. Ia':
{(R,S)-2-Methyl-1,4-dioxaspiro[4,5]dec-2-yl}methylbutyrat,
{(R,S)-2,2,4-Trimethyl-1,3-dioxolan-4-yl}methylbutyrat,
{(S)-2-Methyl-1,4-dioxaspiro[4,5]dec-2-yl}methylbutyrat und
{(S)-2,2,4-Trimethyl-1,3-dioxolan-4-yl}methylbutyrat.
Formel Ib' bzw. Ib'':
{(S)-2-Methyl-1,4-dioxaspiro[4,5]dec-2-yl}metnanol und
[(R)-2-Methyl-1,4-dioxaspiro[4,5]dec-2-yl}methanol.
Formel Ic' bzw. Ic'':
{(R)-2-Methyl-1,4-dioxaspiro[4,5]dec-2-yl}methyltosylat,
{(S)-2-Methyl-1,4-dioxaspiro[4,5]dec-2-yl}methyltosylat.

Aufgrund der Kohlenstoffzahl des Grundgerüstes der Verbindungen I lassen sie sich als optisch aktive C₄-Bausteine bezeichnen.

Die beiden Produkte der erfindungsgemässen Hydrolyse sind der (S)-Alkohol Ib' und der (S)-Ester Ia', wobei das letztere Produkt eine (nicht umgesetzte) Komponente des racemischen Ausgangsmaterials Ia ist. Ausgehend von den beiden isolierten Produkten können die bekannten optisch aktiven Diole der Formeln III und IV hergestellt werden (siehe Reaktionsschemata). Bei dem einen synthetischen Weg handelt es sich um eine konventionelle Veresterung des (S)-Alkohols Ib' zu dessen Alkan- oder Arylsulfonat der Formel Ic', wobei die Inversion S→R stattfindet, gefolgt von einer wiederum unter dem Fachmann geläufigen Reaktionsbedingungen durchgeführten Abspaltung der Schutzgruppe R¹R²C<. Bei dem zweiten synthetischen Weg muss zunächst einmal der (S)-Ester Ia' zum (R)-Alkohol Ib'' hydrolysiert werden, was unter dem Fachmann geläufigen Reaktionsbedingungen erfolgen kann. Danach kann der (R)-Alkohol mittels Veresterung und anschliessender Abspaltung der Schutzgruppe R¹R²C< in das Diol der Formel IV umgewandelt werden, und zwar analog den Reaktionsstufen Ib'→Ic' und Ic'→III. So erfolgt die Veresterung (Ib'→Ic' bzw. Ib''→Ic'') zweckmässigerweise in einem inerten organischen Lösungsmittel, vorzugsweise einen halogenierten aliphatischen Kohlenwasserstoff, insbesondere Methylenchlorid, bei Temperaturen zwischen 0°C und der Raumtemperatur. Das Alkan- bzw. Arylsulfonierungsmittel ist geeigneterweise das entsprechende Sulfonsäurechlorid, wie beispielsweise Methansulfonylchlorid (Mesylchlorid) oder p-Toluolsulfonylchlorid (Tosylchlorid). Zudem ist das Vorhandensein einer Base, wie eines tertiären Amins, vorzugsweise Triäthylamin, vorteilhaft. Auch die anschliessende Abspaltung der Schutzgruppe (Ic'→III bzw. Ic''→IV) wird zweckmässigerweise in einem inerten organischen Lösungsmittel durchgeführt, und zwar vorzugsweise in einem niederen Alkohol, wie Methanol. Die Reaktion erfolgt zudem geeigneterweise in Gegenwart einer katalytischen Menge einer Sulfonsäure, wie p-Toluolsulfonsäure. Eine bevorzugte Methode besteht darin, das man die geschützte Verbindung (Ic' bzw. Ic'') einer Umketalisierung unterwirft. Beispielsweise wird ein 1,4-Dioxaspiro[4,5]decanderivat der Formel Ic' bzw. Ic'' (R¹ und R² bedeuten zusammen Pentamethylen) wiederholt aus Methanol in Gegenwart von p-Toluolsulfonsäure eingedampft, wobei schliesslich das Diol-Produkt III bzw. IV entsteht.

Die Diole der Formeln III und IV sind bekannte Verbindungen (siehe beispielsweise die europäischen Patentpublikationen Nrn. 257.503 und 269.009) und lassen sich nach an sich bekannten Methoden in d-α-Tocopherol umwandeln (siehe beispielweise die zwei obenerwähnten europäischen Patentpublikationen sowie die europäische Patentpublikation Nr. 129.252). Die Ueberführung des S-Diols (Formel IV) in d-α-Tocopherol kann so erfolgen, dass man dieses Diol mit dem aromatischen Ring koppelt, das Produkt einem Ringschluss unterwirft und anschliessend die C₁₅-Seitenkette anfügt, oder man führt die beiden letzten Reaktionsstufen in umgekehrter Reihenfolge durch. Bei Verwendung des R-Diols (Formel III) andererseits koppelt man dieses zunächst einmal mit der C₁₅-Seitenkette, um zum "C₁₉-Baustein" zu gelangen. Dann verknüpft man diesen benzylisch mit dem aromatischen Ring und führt den Ringschluss durch. Die Details für die verschiedenen Reaktionsstufen finden sich in den obenerwähnten Patentpublikationen.

Die erfindungsgemässe Ueberführung in d-α-Tocopherol wird dadurch durchgeführt, dass man entweder den durch die erfindungsgemässe Hydrolyse hergestellten Alkohol der Formel Ib' zum entsprechenden (R)-Alkansulfonat oder (R)-Arylsulfonat der Formel Ic' verestert, die Schutzgruppe R¹R²C< abspaltet, und das resultierende (R)-Diol der Formel III in an sich bekannter Weise in d-α-Tocopherol überführt, oder den bei der Durchführung der erfindungsgemässen enzymatischen Hydrolyse im Ausgangsmaterial verbleibenden (S)-Alkansäureester der Formel Ia' zum entsprechenden (R)-Alkohol der Formel Ib'' hydrolysiert, diesen (R)-Alkohol zum entsprechenden (S)-Alkansulfonat oder (S)-Arylsulfonat der Formel Ic'' verestert, die Schutzgruppe R¹R²C< abspaltet, und das resultierende (S)-Diol der Formel IV in an sich bekannter Weise in d-α-Tocopherol überführt.

Die nachstehenden Beispiele illustrieren die Erfindung.

### Beispiel 1

### Herstellung von {(R,S)-2-Methyl-1,4-dioxaspiro[4,5]dec-2-yl}methylbutyrat

5,62 g (26,7 mMol) (R,S)-2-Methyl-1,4-dioxaspiro[4,5]decan-2-methanol werden in 20 ml Methylenchlorid gelöst. Zur Lösung gibt man unter Rühren 4,57 ml (3,32 g, 32,8 mMol) Triäthylamin, 4,90 ml (4,75 g, 30,0 mMol) Buttersäureanhydrid und eine katalytische Menge 4-Dimethylaminopyridin zu, und anschliessend rührt man das Reaktionsgemisch weitere 5 Stunden bei Raumtemperatur. Das Gemisch wird dann mit Wasser gewaschen, die organische Phase unter vermindertem Druck eingeengt und der Rückstand über Kieselgel 60 (0,040-0,063 mm, 300 g) unter Verwendung von n-Hexan/Aethylacetat (4:1) als Laufmittel chromatographisch gereinigt. Man erhält auf diese Weise 6,40 g (25,0 mMol, 93,5% der theoretischen Ausbeute) des Butyrats als farbloses Oel, das sich durch Gaschromatographie als 99% rein erweist und keine Spuren von Edukt-Alkohol enthält.

### Beispiel 2

### Herstellung von {(R,S)-2,2,4-Trimethyl-1,3-dioxolan-4-yl}methylbutyrat

17,3 g (118,3 mMol) {(R,S)-2,2,4-Trimethyl-1,3-dioxolan-4-yl}methanol werden in 70 ml Methylenchlorid gelöst. Zur Lösung gibt man unter Rühren 23,0 ml (16,7 g, 165,0 mMol) Triäthylamin, 19,4 ml (18,8 g, 118,7 mMol) Buttersäureanhydrid und eine katalytische Menge 4-Dimethyl-aminopyridin zu, und anschliessend rührt man das Reaktionsgemisch weitere 5 Stunden bei Raumtemperatur. Das Gemisch wird dann mit Wasser gewaschen, die organische Phase unter vermindertem Druck eingeengt und der Rückstand über Kieselgel 60 (0,040-0,063 mm) unter Verwendung von n-Hexan/Aethylacetat (3:1) als Laufmittel chromatographisch gereinigt. Man erhält auf diese Weise 23,5 g (108,6 mMol, 91,8 % der theoretischen Ausbeute) des Butyrats als farbloses Oel, das sich durch Gaschromatographie als mehr als 99 % rein erweist und keine Spuren von Edukt-Alkohol enthält.

### Beispiel 3

### Herstellung von {(S)-2-Methyl-1,4-dioxaspiro[4,5]dec-2-yl}methanol (asymmetrische Hydrolyse von {(R,S)-2-Methyl-1,4-dioxaspiro[4,5]dec-2-yl}methylbutyrat)

100 mg (390 µMol) {(RS)-2-Methyl-1,4-dioxaspiro-[4,5]dec-2-yl}methylbutyrat werden in 25 ml 0,1M Natriumchloridlösung und 1 ml 0,1M Natriumphosphatpuffer bei pH 7 emulgiert. Nach Adjustieren des pH-Wertes auf 7,5 (oder 7,0) mit 0,1N Natriumhydroxidlösung wird die Reaktion durch Zugabe einer katalytischen Menge des jeweiligen Enzyms in Gang gebracht. Unter Einrühren von 0,1N Natriumhydroxidlösung wird der pH-Wert bei 7,5 bzw. 7,0 konstant gehalten, und zwar bis zum ca. 50-prozentigen Reaktionsumsatz [nach Zugabe von ca. 195 µMol NaOH (50% Ester-Aequivalente, 1,95 ml)]. Danach wird die Reaktion durch Zugabe von 25 ml Methylenchlorid abgebrochen. Man extrahiert das Reaktionsgemisch mit 25 ml Methylenchlorid, wobei die Phasentrennung durch kurzzeitige Zentrifugation beschleunigt wird, trocknet die organische Phase über wasserfreiem Magnesiumsulfat, dampft das Lösungsmittel ab und appliziert den Rückstand zwecks direkter Bestimmung der Enantiomerenreinheit des Produktalkohols (mittels chiraler Phase: permethyliertes β-Cyclodextrin) auf Kapillar-Gaschromatographie.

Die verwendeten Enzyme, der prozentuale Umsatz, die entsprechende Reaktionszeit sowie die prozentuale Enantiomerenreinheit (ee) des so hergestellten {(S)-2-Methyl-1,4-dioxaspiro[4,5]dec-2-yl}methanols sind in der nachfolgenden Tabelle 1 angegeben.

**Tabelle 1**

| Enzym (eingesetzte Menge*) | Prozentualer Umsatz/Reaktionszeit | Prozentuale ee des Produkt-Alkohols |
|---|---|---|
| Lipase P-30 (200 U) | 48,6/35 Min. | 99 |
| Lipase SAM (85 U) | 48,1/168 Min.** | 99 |
| Lipase PM [Palatase M 1000 L] (100 µl) | ca. 50/40 Min. | 98 |
| Lipase LMM [Lipozym IM 20] | ca. 49 | 91 |
| Lipase LRD (525 U) | 48,8/52 Min. | 98 |
| Lipase D-20 (14 mg) | 49,2/38 Min. | 97 |
| Lipase LRA (1481 U) | 49,5/15 Min. | 97 |
| Lipase CE-5 (23 mg) | 49,0/10 Min. | 98 |
| Lipase F-AP 15 (1008 U) | 47,6/176 Min.** | 95 |
| Lipase M-AP 10 (80 U) | 48,7/168 Min. | 96 |

| | | |
|---|---|---|
| * Menge in mg, µl bzw. U (Menge verwendeter Einheiten gemäss vom Lieferanten deklarierten Angaben) | | |
| ** Reaktion bei pH 7,0 durchgeführt (sonst 7,5) | | |

### Beispiel 4

### Herstellung von {(S)-2-Methyl-1,4-dioxaspiro[4,5]dec-2-yl}methanol (asymmetrische Hydrolyse von {(R,S)-2-Methyl-1,4-dioxaspiro[4,5]dec-2-yl}methylbutyrat, alternative Methode)

Das Verfahren des Beispiels 3 wird wiederholt, allerdings mit den Unterschieden, dass 1,0 g (3,91 mMol) oder 3,0 g (11,73 mMol) statt 100 mg (390 µMol) des Butyrats eingesetzt und 1,0N Natriumhydroxidlösung statt 0,1N Natriumhydroxidlösung als Titriermittel verwendet wird.

Die diesbezüglichen Angaben sind in der nachfolgenden Tabelle 2 aufgeführt:

**Tabelle 2**

| Enzym (eingesetzte Menge***) | Menge eingesetzten Butyrats | Prozentualer Umsatz/Reaktionszeit | Prozentuale ee des Produkt-Alkohols |
|---|---|---|---|
| Lipase P-30 (600 U) | 1,0 g | 47,3/150 Min. | 99,2 |
| Lipase P-30 (1800 U) | 3,0 g | 48,9/200 Min. | 99,1 |
| Lipase PM [Palatase M 1000 L] (300 µl) | 1,0 g | 45,1/154 Min. | 97,3 |
| Lipase PM [Palatase M 1000 L] (1,0 ml) | 3,0 g | 47,2/316 Min. | 96,0 |
| Lipase LRD (1575 U) | 1,0 g | 49,8/184 Min. | 97,7 |
| Lipase LRD (5250 U) | 3,0 g | 48,6/370 Min. | 95,9 |

| | | | |
|---|---|---|---|
| *** Menge in ml,µl bzw. U (Menge verwendeter Einheiten gemäss vom Lieferanten deklarierten Angaben) | | | |

### Beispiel 5

### Herstellung von {(S)-2-Methyl-1,4-dioxaspiro[4,5]dec-2-yl}methanol (asymmetrische Hydrolyse von {(R,S)-2-Methyl-1,4-dioxaspiro[4,5]dec-2-yl}methylbutyrat)

Das Verfahren des Beispiels 3 wird mit Lipase P-30 (200 U) bei pH 7.5 wiederholt, allerdings mit dem Unterschied, dass
- 0,1 M Lithiumrhodanid anstelle von 0,1 M Natriumchlorid (Variante A),
- 0,1 M Glycerin anstelle von 0,1 M Natriumchlorid (Variante B),
- 0,1 M Calciumchlorid anstelle von 0,1 M Natriumchlorid (Variante C),
- 0.1 M Magnesiumchlorid anstelle von 0,1 M Natriumchlorid (Variante D)
   verwendet wird oder
- die wässrige Phase Zusätzlich noch 26 µl Triton X-100 enthält (Variante E).

Die diesbezüglichen Angaben sind in der nachfolgenden Tabelle 3 aufgeführt:

**Tabelle 3**

| Variante | Prozentualer Umsatz | Reaktionszeit | Prozentuale ee des Produkt-Alkohols |
|---|---|---|---|
| A | 49,5 | 41 Min. | 99 |
| B | 49,6 | 48 Min. | 99 |
| C | 49,5 | 36 Min. | 99 |
| D | 49,6 | 29 Min. | 99 |
| E | 49,5 | 59 Min. | 99 |

### Beispiel 6

### Herstellung von {(S)-2,2,4-Trimethyl-1,3-dioxolan-4-yl}-methanol (asymmetrische Hydrolyse von {(R,S)-2,2,4-Trimethyl-1,3-dioxolan-4-yl}methylbutyrat)

Analog dem in Beispiel 3 beschriebenen Verfahren wird 100 mg (462 µMol) {(R,S)-2,2,4-Trimethyl-1,3-dioxolan-4-yl}methylbutyrat zum entsprechenden Methanolderivat hydrolysiert. Zur Bestimmung der Enantiomerenreinheit des Produkt-Alkohols mittels Kapillar-Gaschromatographie (permethyliertes β-Cyclodextrin) wird dieser vorgängig unter Verwendung von Benzoylchlorid in den entsprechenden Benzoesäureester übergeführt.

Die verwendeten Enzyme, der prozentuale Umsatz, die entsprechende Reaktionszeit sowie die prozentuale Enantiomerenreinheit (ee) des so hergestellten {(S)-2,2,4-Trimethyl-1,3-dioxolan-4-yl}methanols sind in der nachfolgenden Tabelle 4 angegeben:

**Tabelle 4**

| Enzym (eingesetzte Menge*) | Prozentualer Umsatz/Reaktionszeit | Prozentuale ee des Produkt-Alkohols |
|---|---|---|
| Lipase P-30 (200 U) | 49.5/26 Min. | 99,6 |
| Lipase SAM (85 U) | 49,5/81 Min. | 99,6 |
| Lipase PM [Palatase M 1000 L] (100 µl) | 49,6/26 Min. | 99,4 |
| Lipase LRD (525 U) | 49,5/28 Min. | 98,2 |
| Lipase D-20 (14 mg) | 49,5/24 Min. | 97,0 |
| Lipase F-AP 15 (1008 U) | 49,5/16 Min. | 97,1 |
| Lipase M-AP 10 (80 U) | 49,5/60 Min. | 96,6 |

| | | |
|---|---|---|
| * Menge in mg,µl bzw. U (Menge verwendeter Einheiten gemäss vom Lieferanten deklarierten Angaben) | | |

### Beispiel 7

### Herstellung von {(S)-2-Methyl-1,4-dioxaspiro[4,5]dec-2-yl}methanol (asymmetrische Hydrolyse von {(R,S)-2-Methyl-1,4-dioxaspiro[4,5]dec-2-yl}methylbutyrat)

5,00 g (19,5 mMol) {(R,S)-2-Methyl-1,4-dioxaspiro-[4,5]dec-2-yl}methylbutyrat werden in 100 ml 0,1M Natriumchloridlösung und 4 ml 0,1M Natriumphosphatlösung bei pH 7 emulgiert (Konzentration an Butyrat ca. 4,6% G/V). Die Reaktion wird durch Zugabe von 74,0 mg Lipase P-30 in Gang gebracht. Unter Einrühren von 1,0N Natriumhydroxidlösung wird der pH-Wert bei 7,0 konstant gehalten, und zwar bis zum 45,6-prozentigen Reaktionsumsatz (Reaktionszeit ca. 2,25 Stunden, Zugabe von 8,90 ml Natriumhydroxidlösung). Danach wird die Reaktion durch Zugabe von 50 ml Methylenchlorid abgebrochen. Man extrahiert das Reaktionsgemisch zweimal mit je 50 ml Methylenchlorid, wobei die Phasentrennung durch kurzzeitige Zentrifugation beschleunigt wird, trocknet die vereinigten organischen Phasen über wasserfreiem Magnesiumsulfat und dampft das Lösungsmittel bei 16 mmHg/Raumtemperatur ab. Dies ergibt 4,44 g eines farblosen Oels, welches über Kieselgel 60 (0,040-0,063 mm, 99 g) unter Verwendung von 500 ml Methylenchlorid, gefolgt von 500 ml Methylenchlorid/Diäthyläther (1:1), als Laufmittel chromatographisch gereinigt wird. Man erhält auf diese Weise 1,80 g (8,56 mMol, 88% der theoretischen Ausbeute) des {(S)-2-Methyl-1,4-dioxaspiro[4,5]dec-2-yl}methanols, >99%ig gaschromatographisch rein, >99%ige Enantiomerenreinheit (ee), [α]₃₆₅ = -20,1° (1% in CHCl₃) bzw. +20,9° (1% in C₂H₅OH).

[Die ebenfalls isolierte Ester-Fraktion ({(S)-2-Methyl-1,4-dioxaspiro[4,5]dec-2-yl}methylbutyrat) kann eingeengt und in alkalischer Lösung zum {(R)-2-Methyl-1,4-dioxaspiro[4,5]dec-2-yl}methanol hydrolysiert werden].

### Beispiel 8

### Herstellung von {(S)-2,2,4-Trimethyl-1,3-dioxolan-4-yl}methanol (asymmetrische Hydrolyse von {(R,S)-2,2,4-Trimethyl-1,3-dioxolan-4-yl}methylbutycat, alternative Methode)

Das Verfahren des Beispiels 6 wird wiederholt, allerdings mit den Unterschieden, dass 1,0 g (4,6 mMol) oder 3,0 g (13,9 mMol) oder 5,0 g (23,1 mMol) statt 100 mg (462 µMol) des Butyrats eingesetzt und 1,0 N Natriumhydroxidlösung als Titriermittel verwendet wird.

Die diesbezueglichen Angaben sind in der nachfolgenden Tabelle 5 aufgeführt:

**Tabelle 5**

| Enzym (eingesetzte Menge***) | Menge eingesetzten Butyrats | Prozentualer Umsatz/Reaktionszeit | Prozentuale dukt-Alkohols |
|---|---|---|---|
| Lipase P-30 (600 U) | 1,0 g | 49,5/28 Min. | 99,4 |
| Lipase P-30 (1800 U) | 3,0 g | 49,3/60 Min. | 99,0 |
| Lipase P-30 (1800 U) | 5,0 g | 48,9/120Min. | 98,8 |
| Lipase PM [Palatase M 1000 L] (300 µl) | 1,0 g | 49,5/90 Min. | 97,3 |
| Lipase LRD (1575 U) | 1,0 g | 49,5/132Min. | 94,4 |

| | | | |
|---|---|---|---|
| *** Menge in µl bzw. U (Menge verwendeter Einheiten gemäss vom Lieferanten deklarierten Angaben) | | | |

### Beispiel 9

### Herstellung von {(S)-2,2,4-Trimethyl-1,3-dioxolan-4-yl}methanol (asymmetrische Hydrolyse von {(R,S)-2,2,4-Trimethyl-1,3-dioxolan-4-yl}methylbutyrat)

5,01 g (23,2 mMol) {(R,S)-2,2,4-Trimethyl-1,3-dioxolan-4-yl}methylbutyrat werden in 25 ml 0,1 M Natriumchloridlösung und 1 ml 0,1 M Natriumphosphatpuffer bei pH 7,5 emulgiert (Konzentration an Butyrat 16,1 % G/V). Die Reaktion wird durch Zugabe von 50 mg Lipase P-30 in Gang gebracht. Unter Einrühren von 1,0 N Natriumhydroxidlösung wird der pH-Wert bei 7,5 konstant gehalten, und zwar bis zum 46,6-prozentigen Reaktionsumsatz (Reaktionszeit 1 Stunde, Zugabe von 10,8 ml Natriumhydroxidlösung). Danach wird die Reaktion durch Zugabe von 50 ml Methylenchlorid abgebrochen. Man extrahiert das Reaktionsgemisch zweimal mit je 50 ml Methylenchlorid, wobei die Phasentrennung durch kurzzeitige Zentrifugation beschleunigt wird, trocknet die vereinigten organischen Phasen über wasserfreiem Magnesiumsulfat und dampft das Lösungsmittel bei 12mbar/30°C ab. Dies ergibt 4,07 g eines Oels, welches über Kieselgel 60 (0,040-0,063 mm, 80 g) unter Verwendung von n-Hexan/Aethylacetat (2:1) als Laufmittel chromatographisch gereinigt wird. Man erhält auf diese Weise 1,33 g (9,11 mMol, 78,6 % der theoretischen Ausbeute) des {(S)-2,2,4-Trimethyl-1,3-dioxolan-4-yl}methanols, >99%ig gaschromatographisch rein, >99%ige Enantiomerenreinheit (ee), [α]₃₆₅= -24,2° (1% in CHCl₃) bzw. [α]₃₆₅= +26,0° (0,5% in Aethanol).

### Beispiel 10

### Herstellung von {(R)-2-Methyl-1,4-dioxaspiro[4,5]dec-2-yl}methyltosylat

Eine Lösung von 1,67 g (7,94 mMol) {(S)-2-Methyl-1,4-dioxaspiro[4,5]dec-2-yl}methanol und 3 ml Triäthylamin in 10 ml Methylenchlorid wird portionenweise mit 1,63 g (8,54 mMol) Tosylchlorid versetzt und das Reaktionsgemich ca. 16 Stunden stehen gelasen. Die resultierende braune Masse wird in 100 ml Methylenchlorid aufgenommen und die Lösung Zweimal mit je 100 ml Wasser gewaschen. Anschliessendes Eindampfen der organischen Phase ergibt 3,1 g eines bräunlichen Oels, welches über Kieselgel 60 (0,040-0,063 mm, 70 g) unter Verwendung von Methylenchlorid als Laufmittel chromatographisch gereinigt wird. Man erhält 2,33 g (6,84 mMol, 86% der theoretischen Ausbeute) des {(R)-2-Methyl-1,4-dioxaspiro-[4,5]dec-2-yl}methyltosylats, 98%ig gaschromatographisch rein, [α]₃₆₅ = -29,0° (1% in CHCl₃).

### Beispiel 11

### Herstellung von (R)-2,3-Dihydroxy-2-methylpropyltoxylat

2,22 g (6,52 mMol) {(R)-2-Methyl-1,4-dioxaspiro[4,5]-dec-2-yl}methyltosylat werden wiederholt aus Methanol in Gegenwart einer katalytischen Menge p-Toluolsulfonsäure eingedampft, und der Rückstand wird schliesslich über Kieselgel 60 (0,040-0,063 mm, 55 g) unter Verwendung von Diäthyläther/Methylenchlorid (1:2) als Laufmittel chromatographisch gereinigt. Man erhält 829 mg (3,18 mMol, 49% der theoretischen Ausbeute) des (R)-2,3-Dihydroxy-2-methylpropyltosylats, 98%ig gaschromatographisch rein, [α]₃₆₅ = -19,9° (1% in CHCl₃).

### Beispiel 12

### Herstellung von (R)-2,3-Dihydroxy-2-methylpropyltosylat

1,42 g (4,74 mMol) {(R)-2,2,4-Trimethyl-1,3-dioxolan-4-yl}methyltosylat werden zweimal aus 100 ml Methanol in Gegenwart einer katalytischen Menge p-Toluolsulfonsäure bei 16 mbar/40°C eingedampft, und der Rückstand wird schliesslich über Kieselgel 60 (0,040-0,063 mMol, 20 g) unter Verwendung von Methylenchlorid/Diäthyläther (1:1) als Laufmittel chromatographisch gereinigt. Man erhält 907 mg (3,49 mMol, 74% der theoretischen Ausbeute) des (R)-2,3-Dihydroxy-2-methylpropyltosylats, 99%ig gaschromatographisch rein, [α]₃₆₅= -19,0° (1% in CHCl₃).

### Beispiel 13

### Herstellung von (R)-α-Methyl-2-oxiranmethanol

816 mg (3,13 mMol) (R)-2,3-Dihydroxy-2-methylpropyltosylat werden in 50 ml Diäthyläther gelöst, und die Lösung wird portionenweise mit 1,08 g (9,68 mMol) Natrium-tert.-butylat versetzt. Nach 3-stündigem Rühren gibt man 100 µl (5,55 mMol) Wasser zu und rührt weitere 30 Minuten. Dann wird das entstandene Kaliumtosylat abfiltriert und das Filtrat vorsichtig eingeengt (Epoxid leichtflüchtig). Der Rückstand wird über Kieselgel 60 (0,040-0,063 mm, 20 g) unter Verwendung von n-Pentan/Diäthyläther (in eine Richtung allmählich wechselndes Verhältnis der beiden Komponenten) als Laufmittel chromatographisch gereinigt. Nach vorsichtigem Einengen der Fraktion erhält man 72 mg an rohem (R)-α-Methyl-2-oxiranmethanol. Die Bestimmung der Enantiomerenreinheit mittels Kapillar-Gaschromatographie (chirale permethylierte β-Cyclodextrin-Phase) ergibt 99%ige ee des (R)-Isomeren (Referenz-Vergleich).

Dies bedeutet, dass der unter der Einwirkung des Enzyms produzierte Alkohol (siehe Beispiele 3-9 und 15) die S-Konfiguration besitzt, ebenfalls der nicht umgesetzte Ester.

### Beispiel 14

### Herstellung von {(S)-2,2,4-Trimethyl-1,3-dioxolan-4-yl}methanol (asymmetrische Hydrolyse von {(R,S)-2,2,4-Trimethyl-1,3-dioxolan-4-yl}methylbutyrat unter Verwendung eines immobilisierten Enzyms)

(i) Partielle Reinigung von Lipase P-30 Die Reinigung erfolgt nach der Methode von M. Sugiura et al. [Biochim. Biophys. Acta 488, 353-358 (1977)]: Man nimmt 12,5 g Lipase P-30 in 100 ml Natriumacetatpuffer (10 mMol, pH 7,0) auf und rührt das Gemisch eine halbe Stunde bei 4°C. Der ungelöste Anteil wird durch Zentrifugation entfernt (43500 g, 20 Min., 5°C) und die verbleibende Lösung auf 100 ml mit einer weiteren Menge Puffer verdünnt, wobei die Enzym-Aktivität 2600 U/ml beträgt [zur Messung dieser Aktivität werden 250 µl Tributyrin in 25 ml 0,1M Natriumchloridlösung und 1 ml 0,1M Natriumphosphatpuffer (pH 7.0) emulgiert, und die Reaktion wird durch Zugabe der Enzymprobe in Gang gebracht; man überwacht den Reaktionsablauf unter Beibehaltung eines pH-Wertes von 7,0 durch Zugabe von 0,1N Natriumhydroxidlösung]. Man gibt zur Lösung innerhalb von 30 Minuten 20,9 g Ammoniumsulfat (35%ige Sättigung) und rührt die so gebildete Suspension weitere 2,5 Stunden bei 0°C. Dann wird die Suspension wie oben beschrieben zentrifugiert und das Sediment (ca. 1 Prozent der Enzym-Aktivität im Ueberstand) in einer kleinen Menge Natriumphosphatpuffer (50 mMol, pH 7,5) gelöst und innert 16 Stunden gegen 4 x 1 l desselben Puffers bei 4°C dialysiert [Spektra/Por, Ausschlussgrenze des Molgewichts 3500 (Spectrum Medical Industries, Los Angeles, Ca., USA)]. Dies ergibt 14,7 ml einer konzentrierten Lösung von Lipase P-30 (1,33·10⁴ U/ml; Enzym-Aktivität wie oben beschrieben gemessen).
(ii) Immobilisation von Lipase P-30 Man verdünnt 500 µl der Lipase-Lösung viermal mit dem oben erwähnten Phosphatpuffer und gibt 500 mg Eupergit C-Kügelchen (Röhm, Weiterstadt, BRD) zu. Dann wird die Suspension 63 Stunden bei Raumtemperatur leicht geschüttelt, und anschliessend werden die Kügelchen abfiltriert,mit 0,1M Natriumchloridlösung gewaschen (ca. 0,1 Prozent der Enzym-Aktivität im Filtrat) und 15 Minuten bei 16 mbar getrocknet. Auf diese Weise erhält man 1,36 g feuchte Kügelchen (234 U/g, diese Enzym-Aktivität wie oben beschrieben gemessen; einige Tage bei 4°C gelagert).
(iii) Asymmetrische Hydrolyse 10,0 g (46,2 mMol) {(RS)-2,2,4-Trimethyl-1,3-dioxolan-4-yl}methylbutyrat werden in 185 ml 0,1M Natriumchloridlösung und 5 ml 0,1M Natriumphosphatpuffer (pH 7,5) emulgiert. Nach Adjustieren des pH-Wertes auf 7,5 mit 1,0N Natriumhydroxidlösung wird die Reaktion durch Zugabe von 500 mg feuchten Eupergit-Kügelchen in Gang gebracht. Unter Einrühren von 1,0N Natriumhydroxidlösung wird der pH-Wert bei 7,5 konstant gehalten. Nach Zugabe von insgesamt 22,2 ml (22,2 mMol; 48-prozentiger Reaktionsumsatz nach 6,9 Stunden) Titriermittel werden die Enzym-Kügelchen abfiltriert und fünfmal mit je 10 ml 0,1M Natriumchloridlösung gewaschen. Das Filtrat wird mit der ersten Waschlösung vereinigt und viermal mit je 200 ml Methylenchlorid extrahiert. Die vereinigten organischen Phasen werden dann über wasserfreiem Natriumsulfat getrocknet und bei 600 mbar/40°C eingeengt. Man unterwirft den Rückstand einer Säulenchromatographie unter Verwendung von Kieselgel (Silica gel 60, 0,040-0,063 mm) und n-Hexan/Aethy lacetat (2:1). Man erhält auf diese Weise 3,02 g (20,7 mMol, 44,7% der theoretischen Ausbeute) der Titelverbindung, die sich durch Gaschromatographie als mehr als 95% rein erweist und eine prozentuale ee von mehr als 99% aufweist.

## Patentansprüche

1. Verfahren zur Herstellung einer Verbindung der allgemeinen Formel 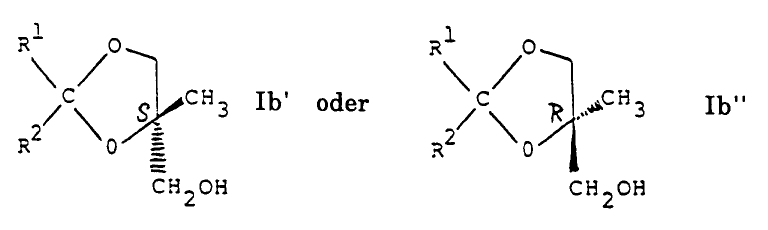 worin R¹ und R² unabhängig voneinander Methyl oder Aethyl, oder zusammen Pentamethylen bedeuten,
dadurch gekennzeichnet, dass man einen racemischen Alkansäureester der allgemeinen Formel 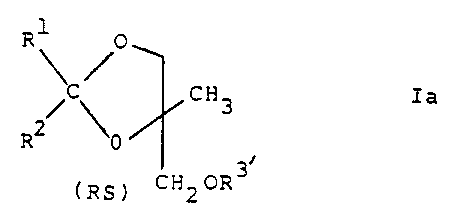 worin R¹ und R² die oben angegebenen Bedeutungen besitzen und R^{3'} C₂₋₉-Alkanoyl bedeutet,
unter Verwendung eines Enzyms der Unterklasse Carboxylesterasen (EC 3.1.1.1), Triacylglycerinlipasen (EC 3.1.1.3), Cholesterinesterasen (EC 3.1.1.13) oder Diacylglycerinlipasen (EC 3.1.1.34) zur Verbindung der allgemeinen Formel Ib' hydrolysiert bzw. den bei der Durchführung der enzymatischen Hydrolyse nicht umgesetzten (S)-Alkansäureester der allgemeinen Formel 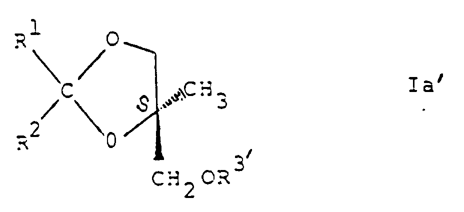 worin R¹, R² und R^{3'} die oben angegebenen Bedeutungen besitzen,
zum entsprechenden (R)-Alkohol der allgemeinen Formel 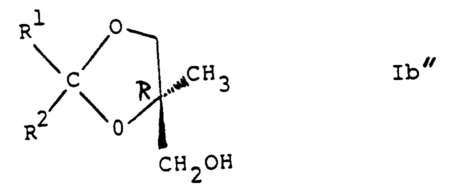 worin R¹ und R² die oben angegebenen Bedeutungen besitzen,
hydrolysiert.

2. Verfahren nach Anspruch 1, dadurch gekennzeichnet, dass als Enzym eine Lipase aus Pseudomonas fluorescens, Mucor miehei, Rhizopus delemar, Rhizopus arrhizus, Humicola lanuginosa, Rhizopus javanicus oder Mucor javanicus verwendet wird.

3. Verfahren nach Anspruch 1 oder 2, dadurch gekennzeichnet, dass man {(RS)-2-Methyl-1,4-dioxaspiro[4,5]dec-2-yl}methylbutyrat zum {(S)-2-Methyl-1,4-dioxaspiro-[4,5]dec-2-yl}methanol enzymatisch hydrolysiert.

4. Verfahren nach Anspruch 1 oder 2, dadurch gekennzeichnet, dass man {(RS)-2,2,4-Trimethyl-1,3-dioxolan-4-yl}methylbutyrat zum {(S)-2,2,4-Trimethyl-1,3-dioxolan-4-yl}methanol enzymatisch hydrolysiert.

5. Verfahren nach einem der Ansprüche 1 bis 4, dadurch gekennzeichnet, dass in der Formel Ia R¹ und R² beide Methyl oder zusammen Pentamethylen bedeuten.

6. Verfahren nach einem der Ansprüche 1 bis 5, dadurch gekennzeichnet, dass man die Hydrolyse des racemischen Alkansäureesters der allgemeinen Formel Ia nach Erreichen eines 50-prozentigen Reaktionsumsatzes abbricht.

7. Verwendung des Verfahrens gemäss einem der Ansprüche 1 bis 6 zur Herstellung von d-α-Tocopherol, dadurch gekennzeichnet, dass man den so hergestellten (S)-Alkohol der Formel Ib' bzw. (R)-Alkohol der Formel Ib'' zum entsprechenden (R)-Alkansulfonat oder (R)-Arylsulfonat verestert, die Schutzgruppe R¹R²C< abspaltet und das resultierende (R)- bzw. (S)-Diol in an sich bekannter Weise in d-α-Tocopherol überführt.

8. Verbindungen der allgemeinen Formel 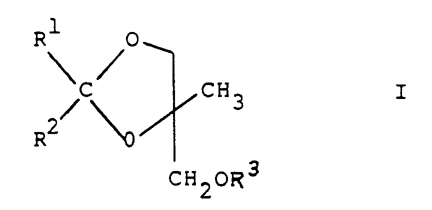 worin
R¹ und R² unabhängig voneinander Methyl oder Aethyl, oder zusammen Pentamethylen
und
R³ Wasserstoff, C₂₋₉-Alkanoyl, Alkansulfonyl oder Arylsulfonyl bedeuten, wobei, falls R¹ und R² beide für Methyl stehen, R³ verschieden von Wasserstoff oder Arylsulfonyl ist, und falls R¹ und R² zusammen für Pentamethylen und R³ für Wasserstoff stehen, die Verbindung I chiral ist.

9. Verbindungen nach Anspruch 8 der allgemeinen Formel 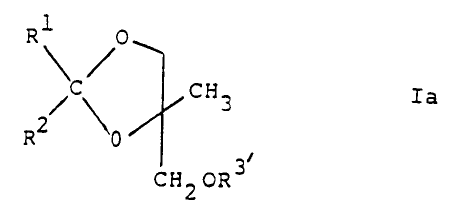 worin R¹ und R² die in Anspruch 8 angegebenen Bedeutungen besitzen und R^{3'} C₂₋₉-Alkanoyl bedeutet.

10. Verbindungen nach Anspruch 9 in der (S)-Enantiomerenform.

11. Verbindungen nach Anspruch 8 der allgemeinen Formel 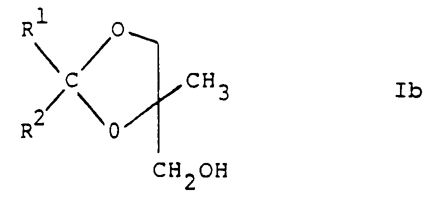 worin R¹ und R² unabhängig voneinander Methyl oder Aethyl, oder zusammen Pentamethylen bedeuten, wobei R¹ und R² nicht beide für Methyl stehen, und falls R¹ und R² zusammen für Pentamethylen stehen, die Verbindung Ib chiral ist.

12. Verbindungen nach Anspruch 11 in der (S)- oder (R)-Enantiomerenform.

13. Verbindungen nach Anspruch 8 der allgemeinen Formel 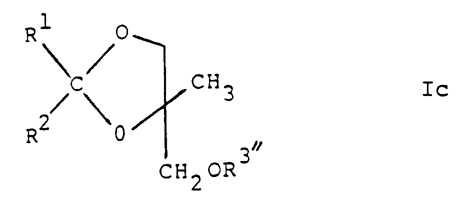 worin R¹ und R² die in Anspruch 8 angegebenen Bedeutungen besitzen und R^{3''} Alkan- oder Arylsulfonyl bedeutet, wobei, falls R¹ und R² beide für Methyl Stehen, R^{3''} verschieden von Arylsulfonyl ist.

14. Verbindungen nach Anspruch 13 in der (R)- oder (S)-Enantiomerenform.

15. Eine Verbindung nach Anspruch 8, ausgewählt aus
{(R,S)-2-Methyl-1,4-dioxaspiro[4,5]dec-2-yl}methylbutyrat,
{(R,S)-2,2,4-Trimethyl-1,3-dioxolan-4-yl}methylbutyrat,
{(S)-2-Methyl-1,4-dioxaspiro[4,5]dec-2-yl}methylbutyrat,
{(S)-2,2,4-Trimethyl-1,3-dioxolan-4-yl}methylbutyrat,
{(S)-2-Methyl-1,4-dioxaspiro[4,5]dec-2-yl}methanol,
{(R)-2-Methyl-1,4-dioxaspiro[4,5]-dec-2-yl}methanol,
{(R)-2-Methyl-1,4-dioxaspiro[4,5]dec-2-yl}methyltosylat, und
{(S)-2-Methyl-1,4-dioxaspiro[4,5]dec-2-yl}methyltosylat.

## Claims

1. A process for the manufacture of a compound of the general formula 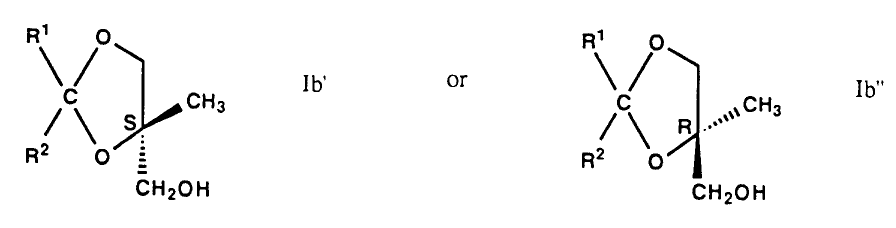 wherein R¹ and R² each independently signify methyl or ethyl or together signify pentamethylene,
characterized by hydrolyzing a racemic alkanoic acid ester of the general formula 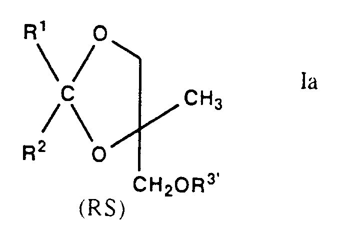 wherein R¹ and R² have the significances given above and R^{3'} signifies C₂₋₉-alkanoyl,
to the compound of general formula Ib' using an enzyme of the sub-class carboxyl esterases (EC 3.1.1.1), triacylglycerol lipases (EC 3.1.1.3), cholesterol esterases (EC 3.1.1.13) or diacylglycerol lipases (EC 3.1.1.34) or hydrolyzing the (S)-alkanoic acid ester of the general formula 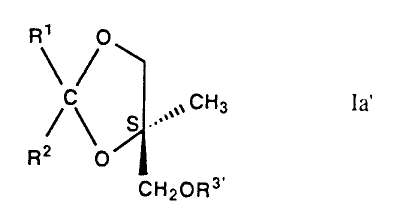 wherein R¹, R² and R^{3'} have the significances given above,
which does not react when the enzymatic hydrolysis is carried out, to the corresponding (R)-alcohol of the general formula 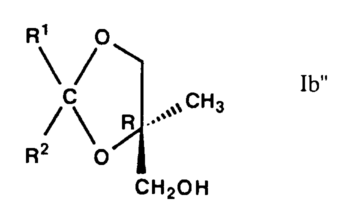 wherein R¹ and R² have the significances given above.

2. A process according to claim 1, characterized in that a lipase from Pseudomonas fluorescens, Mucor miehei, Rhizopus delemar, Rhizopus arrhizus, Humicola lanuginosa, Rhizopus javanicus or Mucor javanicus is used as the enzyme.

3. A process according to claim 1 or 2, characterized in that {(RS)-2-methyl-1,4-dioxaspiro[4,5]dec-2-yl}methyl butyrate is enzymatically hydrolyzed to {(S)-2-methyl-1,4-dioxaspiro[4,5]dec-2-yl}methanol.

4. A process according to claim 1 or 2, characterized in that {(RS)-2,2,4-trimethyl-1,3-dioxolan-4-yl}methyl butyrate is enzymatically hydrolyzed to {(S)-2,2,4-trimethyl-1,3-dioxolan-4-yl}methanol.

5. A process according to any one of claims 1 to 4, characterized in that in formula Ia R¹ and R² both signify methyl or together signify pentamethylene.

6. A process according to any one of claims 1 to 5, characterized in that the hydrolysis of the racemic alkanoic acid ester of general formula Ia is interrupted after a 50 per cent reaction conversion has been achieved.

7. The use of the process in accordance with any one of claims 1 to 6 for the manufacture of d-α-tocopherol, characterized by esterifying the thus-manufactured (S)-alcohol of formula Ib' or (R)-alcohol of formula 1b'' to the corresponding (R)-alkanesulphonate or (R)-arylsulphonate, cleaving off the protecting group R¹R²C< and converting the resulting (R)- or (S)-diol into d-α-tocopherol in a manner known per se.

8. Compounds of the general formula 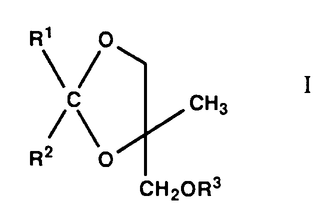 wherein
R¹ and R² each independently signify methyl or ethyl or together signify pentamethylene
and
R³ signifies hydrogen, C₂₋₉-alkanoyl, alkanesulphonyl or arylsulphonyl, whereby R³ is different from hydrogen or arylsulphonyl when R¹ and R² both stand for methyl and compound I is chiral when R¹ and R² together stand for pentamethylene and R³ stands for hydrogen.

9. Compounds according to claim 8 of the general formula 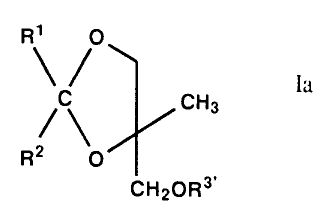 wherein R¹ and R² have the significances given in claim 8 and R^{3'} signifies C₂₋₉-alkanoyl.

10. Compounds according to claim 9 in the (S)-enantiomeric form.

11. Compounds according to claim 8 of the general formula 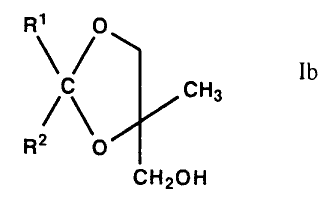 wherein R¹ and R² each independently signify methyl or ethyl or together signify pentamethylene, whereby R¹ and R² do not both stand for methyl,and compound Ib is chiral when R¹ and R² together stand for pentamethylene.

12. Compounds according to claim 11 in the (S)- or (R)-enantiomeric form.

13. Compounds according to claim 8 of the general formula 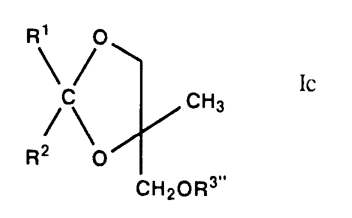 wherein R¹ and R² have the significances given in claim 8 and R^{3''} signifies alkanesulphonyl or arylsulphonyl, whereby R^{3''} is different from arylsulphonyl when R¹ and R² both stand for methyl.

14. Compounds according to claim 13 in the (R)- or (S)-enantiomeric form.

15. A compound according to claim 8, selected from
{(R,S)-2-methyl-1,4-dioxaspiro[4,5]dec-2-yl}methyl butyrate.
{(R,S)-2,2,4-trimethyl-1,3-dioxolan-4-yl}methyl butyrate,
{(S)-2-methyl-1,4-dioxaspiro[4,5]dec-2-yl}methyl butyrate,
{(S)-2,2,4-trimethyl-1,3-dioxolan-4-yl}methyl butyrate,
{(S)-2-methyl-1,4-dioxaspiro[4,5]dec-2-yl}methanol,
{(R)-2-methyl-1,4-dioxaspiro[4,5]-dec-2-yl}methanol,
{(R)-2-methyl-1,4-dioxaspiro[4,5]dec-2-yl}methyl tosylate and
{(S)-2-methyl-1,4-dioxaspiro[4,5]dec-2-yl}methyl tosylate.

## Revendications

1. Procédé de préparation d'un composé de formule générale: 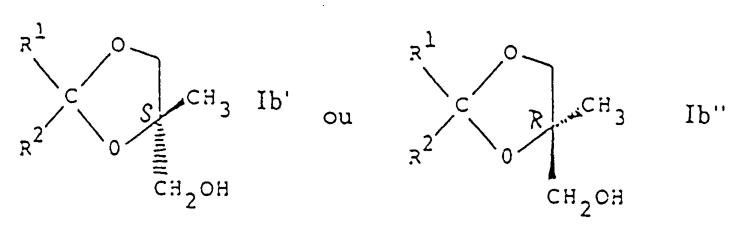 dans laquelle R¹ et R² représentent indépendamment l'un de l'autre un méthyle ou un étyle, ou ensemble un pentaméthylène,
caractérisé en ce qu'on hydrolyse un ester d'acide alcanoïque racémique de formule générale 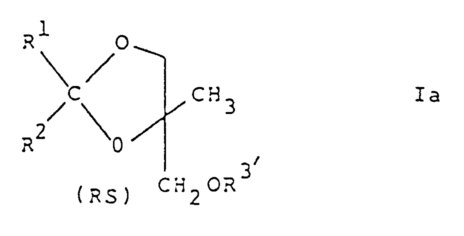 dans laquelle R¹ et R² ont les significations données ci-dessus et R^{3'} représente un alcanoyle en C₂ à C₉,
en utilisant un enzyme de la sous-classe des carboxylestérases (EC 3.1.1.1), des triacylglycérine lipases (EC 3.1.1.3), des cholestérine estérases (EC 3.1.1.13) ou des diacylglycérine lipases (EC 3.1.1.34) pour donner un composé de formule générale Ib', ou en ce qu'on hydrolyse l'ester d'acide (S) alcanoïque de formule générale 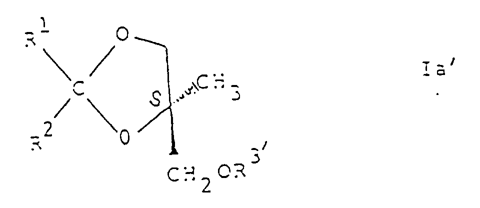 dans laquelle R¹, R² et R³ ont les significations données ci-dessus,
n'ayant pas réagi lors de la réalisation de l'hydrolyse enzymatique,
pour donner le (R)-alcool correspondant de formule générale 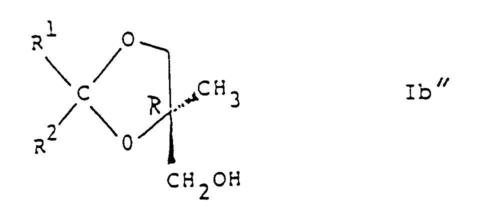 dans laquelle R¹ et R² ont les significations données ci-dessus.

2. Procédé selon la revendication 1, caractérisé en ce qu'on utilise comme enzyme une lipase provenant de Pseudomonas fluorescens, Mucor miehei, Rhizopus delemar, Rhizopus arrhizus, Humicola lanuginosa, Rhizopus -javanicus ou Mucor javanicus.

3. Procédé selon la revendication 1 ou 2, caractérisé en ce qu'on hydrolyse par un moyen enzymatique le {(RS)-2-méthyl-1,4-dioxaspiro[4,5]déc-2-yl}méthylbutyrate en {(S)-2-méthyl-1,4-dioxaspiro [4,5]déc-2-yl}méthanol.

4. Procédé selon la revendication 1 ou 2, caractérisé en ce qu'on hydrolyse par un moyen enzymatique le {(RS)-2,2,4-triméthyl-1,3-dioxolan-4-yl}méthylbutyrate en {(S)-2,2,4-triméthyl-1,3-dioxolan-4-yl}méthanol.

5. Procédé selon l'une des revendications 1 à 4, caractérisé en ce que dans la formule Ia R¹ et R² représentent tous deux un méthyle ou ensemble un pentaméthylène.

6. Procédé selon l'une des revendications 1 à 5, caractérisé en ce qu'on interrompt l'hydrolyse de l'ester d'acide alcanoïque racémique de formule générale Ia après obtention d'un degré de transformation de 50 %.

7. Application du procédé selon l'une des revendications 1 à 6 à la préparation de d-α-tocophérol, caractérisée en ce qu'on estérifie le (S)-alcool de formule Ib' ou le (R)-alcool de formule Ib'' ainsi obtenu pour donner le (R)-alcanesulfonate ou (R)-arylsulfonate correspondant, on ce qu'on sépare le groupe protecteur R¹R²C< et en ce qu'on transforme de façon connue le (R)-ou (S)-diol résultant en le d-α-tocophérol.

8. Composés de formule générale 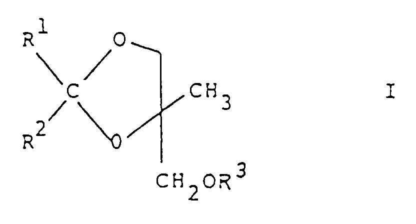 dans laquelle
R¹ et R² représentent indépendamment l'un de l'autre un méthyle ou un éthyle, ou ensemble un pentaméthylène
et
R³ représente un hydrogène, alcanoyle en C₂ à C₉, alcanesulfonyle ou arylsulfonyle, où si R¹ et R² représentent tous deux un méthyle, R³ est différent d'un hydrogène ou arylsulfonyle et, si R¹ et R² représentent ensemble un pentaméthylène et R³ un hydrogène, le composé I est chiral.

9. Composés selon la revendication 8 de la formule générale 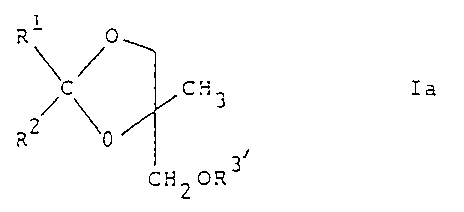 dans laquelle R¹ et R² ont la signification donnée dans la revendication 8 et R^{3'} représente un alcanoyle en C₂ à C₉.

10. Composés selon la revendication 9, sous forme d'énantiomère (S).

11. Composés selon la revendication 8, de formule générale 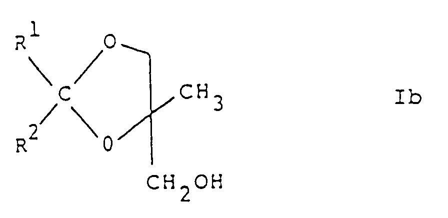 dans laquelle R¹ et R² représentent indépendamment l'un de l'autre un méthyle ou un éthyle, ou ensemble un pentaméthylène où R¹ et R² ne représentent pas tous deux un méthyle, et si R¹ et R² représentent ensemble un pentaméthylène, le composé Ib est chiral.

12. Composés selon la revendication 11 sous forme d'énantiomère (S) ou (R).

13. Composés selon la revendication 8, de formule générale 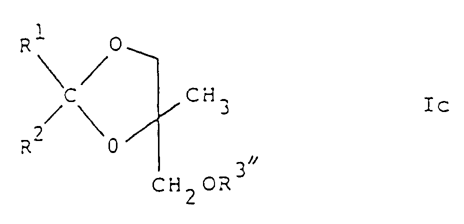 dans laquelle R¹ et R² ont les significations données dans la revendication 8 et R^{3''} représente un alcane- ou aryl-sulfonyle, où, si R¹ et R² représentent tous deux un méthyle, R^{3''} est différent d'un arylsulfonyle.

14. Composés selon la revendication 13 sous forme d'énantiomère (R) ou (S).

15. Composés selon la revendication 8, choisis parmi les suivants:
{(R,S)-2-méthyl-1,4-dioxaspiro[4 5]déc-2-yl}méthylbutyrate
{(R,S)-2,2,4-triméthyl-1,3-dioxolan-4-yl}méthylbutyrate
{(S)-2-méthyl-1,4-dioxaspiro[4,5]déc-2-yl}méthylbutyrate
{(2)-2,2,4-triméthyl-1,3-dioxolan-4-yl}méthylbutyrate
{(S)-2-méthyl-1,4-dioxaspiro[4,5]déc-2-yl}méthanol
{(R)-2-méthyl-1,4-dioxaspiro[4,5]-déc-2-yl}méthanol
{(R)-2-méthyl-1,4-dioxaspiro[4,5]déc-2-yl}méthyltosylate et
{(S)-2-méthyl-1,4-dioxaspiro[4,5]déc-2-yl}méthyltosylate.
